(19) Europäisches Patentamt
European Patent Office
Office européen des brevets

(11) Veröffentlichungsnummer: **0 153 634**
**B1**

# (12) EUROPÄISCHE PATENTSCHRIFT

(45) Veröffentlichungstag der Patentschrift: **23.08.89**

(51) Int. Cl.⁴: **C 12 P 19/44**, C 07 H 15/04 // (C12P19/44, C12R1:38)

(21) Anmeldenummer: **85101344.1**

(22) Anmeldetag: **08.02.85**

(54) **Verfahren zur biotechnischen Herstellung von Rhamnolipiden und Rhamnolipide mit nur einem beta-Hydroxidecancarbonsäurerest im Molekül.**

(30) Priorität: **17.02.84 DE 3405664**

(43) Veröffentlichungstag der Anmeldung: **04.09.85 Patentblatt 85/36**

(45) Bekanntmachung des Hinweises auf die Patenterteilung: **23.08.89 Patentblatt 89/34**

(84) Benannte Vertragsstaaten: **AT CH FR GB IT LI NL**

(56) Entgegenhaltungen:
**FR-A-2 110 410**

**CHEMICAL ABSTRACTS, Band 86, Nr. 15, 11. April 1977, Seite 345, Nr. 104438r, Columbus, Ohio, US; M. YAMAGUCHI et al.: "Microbial production of sugar-lipids"**
**CHEMICAL ABSTRACTS, Band 75, Nr. 9, 30. August 1971, Seite 327, Nr. 62168t, Columbus, Ohio, US; K. HISATSUKA et al.: "Formation of rhamnolipid by Pseudomonas aeruginosa and its function in hydrocarbon fermentation"**

(73) Patentinhaber: **WINTERSHALL AKTIENGESELLSCHAFT, Postfach 10 40 20 Friedrich- Ebert- Strasse 160, D-3500 Kassel (DE)**

(72) Erfinder: **Wagner, Fritz, Prof. Dr., Hohe Wiese 2, D-3301 Stöckheim (DE)**
Erfinder: **Syldatk, Christoph, Dr., Ernst v. Harnackstieg 24, D-3200 Hildesheim (DE)**
Erfinder: **Matulowic, Uwe, Rathenowstrasse 26, D-3300 Braunschweig (DE)**
Erfinder: **Hofmann, Hans- Jürgen, Dr., Stoppelmarkt 2A, D-2848 Vechta (DE)**
Erfinder: **Sewe, Kai- Udo, Dr., Zuckmayerstrasse 8, D-2847 Barnstorf (DE)**
Erfinder: **Lindörfer, Walter, Dr., Christian-Beyerstrasse 16, D-3500 Kassel (DE)**

(74) Vertreter: **Karau, Wolfgang Dr., BASF Aktiengesellschaft Carl- Bosch- Strasse 38, D-6700 Ludwigshafen (DE)**

**EP 0 153 634 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Anionische Rhamnolipide werden bekanntlich von wachsenden Submerskulturen von Mikroorganismen, insbesondere der Pseudomonas-Gattung, gebildet.

So entsteht beispielsweise bei der Züchtung von Pseudomonas pyocyanea in einem Casein/Glucosemedium nach den Angaben von Bergström und anderen in "Arkiv för Kemi, Mineralogi och Geologi", Bd. 23 A (1946) Nr. 13, Seiten 1 bis 12, eine "pyolipic acid", die als saures Lipid bezeichnet ist.

Jarvis und andere berichten in "J. Amer. Chem. Soc.", 71 (1949), Seiten 4124 bis 4126 über ein anionisches Rhamnolipid-(I), das als 2-O-α-L-Rhamnopyranosyl-α-L-rhamnopyranosyl-β-hydroxidecanoyl-β-hydroxidecansäure bezeichnet ist und demnach folgende Strukturformel hat:

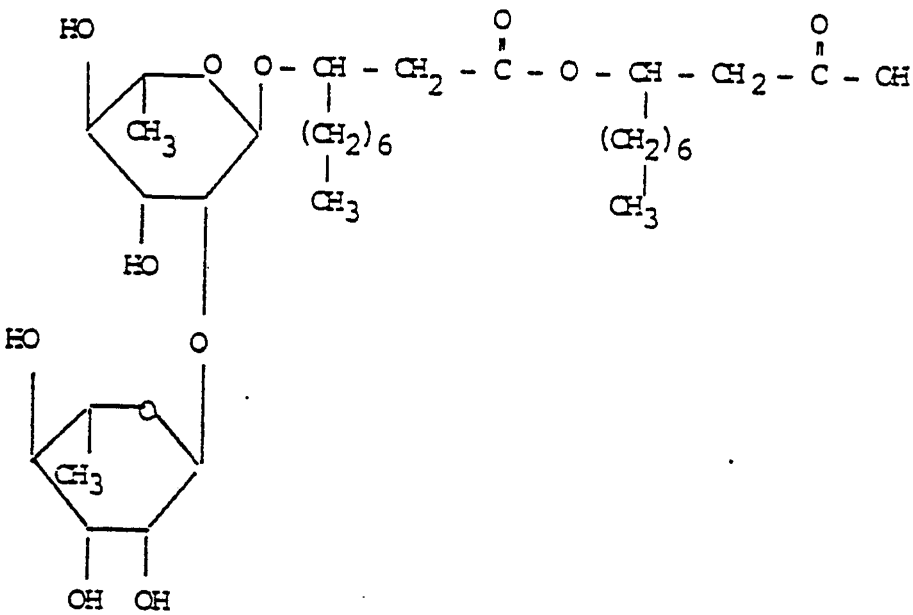

Hisatsuka und andere beschreiben in "Agr. Biol. Chem.", Vol. 5 (1971), Nr. 5, Seiten 686 bis 692 die Bildung von Rhamnolipiden durch Pseudomonas aeruginosa und ihre Wirkung bei der Fermentierung von Kohlenwasserstoffen. Sie erhielten mit Pseudomonas aeruginosa auf n-Paraffinen als Kohlenstoff- und Energiequelle das Rhamnolipid (I). Sie stellten fest, daß dieses Rhamnolipid (I) bereits in verhältnismäßig geringen Konzentrationen das Wachstum von Pseudomonas aeruginosa in einem n-Hexadecanmedium sehr stark fördert. Diese hier durch das Rhamnolipid (I) bewirkte Wachstumsförderung konnte bei anderen Kohlenwasserstoffe verwertenden Mikroorganismen nicht festgestellt werden.

Itoh und andere gehen in ihrer Veröffentlichung in "The Journ. of Antibiotics", Vol. 24 (1971), Seiten 855 bis 859 von diesem Stand der Technik aus und verweisen darauf, daß die genaueren Kenntnisse der Biosynthese des Rhamnolipids (I) Veranlassung dazu gibt, die Entstehung noch eines anderen Rhamnolipids zu vermuten, das eine Vorstufe des Rhamnolipids (I) darstellt. Sie fanden, daß aus einer auf n-Paraffin wachsende Kultur von Pseudomonas aeruginosa außer dem bekannten Rhamnolipid (I), das sie als Rhamnolipid R-1 bezeichneten, noch ein weiteres Rhamnolipid R-2 isoliert werden konnte, das als L-α-Rhamnopyranosyl-β-hydroxidecanonyl-β-hydroxidecanoat identifiziert werden konnte und als Vorstufe des Rhamnolipids (I) erkannten.

Hierüber und mit gleichen Definitionen berichten Itoh und andere auch in "Agr. Biol. Chem.", Vol. 36 (1972), Seiten 2233 bis 2235. Sie berichten weiter, daß Mutanten von Pseudomonas aeruginosa, die die Fähigkeit verloren haben, n-Paraffin als Nährstoff zu nutzen, auf n-Paraffinen normal wachsen, wenn der Kultursuspension Rhamnolipide zugesetzt werden. Sie schließen daraus, daß die Mutanten die enzymatische Aktivität zur Nutzung von n-Paraffinen als Nährstoffquelle nicht verloren haben und die Rhamnolipide eine wesentliche Notwendigkeit für die Verarbeitung von n-Paraffinen durch Pseudomonas aegurinosa-Mikroorganismen sind.

Cooper und andere verweisen in "Microbial Degradation of Polluants in Maine Environments", (1979), Seiten 231 bis 240, in einem Bericht über Rhamnolipide auf die vorerwähnten Arbeiten und geben die wörtliche Bezeichnung der Rhamnolipide in Übereinstimmung mit Itoh und anderen l.c. (1971) wieder. Allerdings geben sie zu dem Rhamnolipid, das als α-L-rhamnopyranosyl-β-hydroxidecanoyl-β-hydroxidecanoat angesprochen ist, unzutreffend eine Strukturformel an, die nur einen Rhamnoserest und auch nur einen Hydroxidecansäurerest aufweist. Der gleiche Fehler findet sich in der Veröffentlichung von Cooper und anderen in "Advances in Applied Microbiol.", Vol. 26 (1980), Seiten 229 bis 253, auf Seiten 234 und 235.

Aus der DE-OS-2 150 375 ist ein Verfahren zur biotechnischen Herstellung rhamnosehaltiger Glycolipide bekannt, nachdem ein derartiges Glycolipide produzierendes Bakterium der Gattung Pseudomonas in einem wäßrigen, mindestens eine assimilierbare Kohlenstoffquelle enthaltenden Nährmedium bei pH-Werten von 4 bis 10 und Temperaturen von 25 bis 37°C aerob gezüchtet wird und die Glycolipide aus der Kulturbrühe isoliert

werden. Dieses Verfahren sollte die Aufgabe lösen, Glycolipide in hohen Ausbeuten zu erhalten. Nach den Beispielen dieser DE-OS werden folgende Ausbeuten mit den genannten Pseudomonasarten auf der ihnen zugeordneten C-Basis erhalten:

| Pseudomonasart | | C-Quelle | Rhamnolipid-Ausbeute in g/l Kulturbrühe |
|---|---|---|---|
| aeruginosa | ATCC 15246 | n-Paraffin | 2,4 |
| fluorescens | ATCC 15453 | n-Paraffin | 5,0 |
| aeruginosa | ATCC 10145 | Glycerin | 0,87 |
| aeruginosa | ATCC 10145 | n-Paraffin | 9,7 |
| aeruginosa | ATCC 15246 | Glucose | 5,6 |
| fluorescens | ATCC 15453 | Glycerin | 3,3 |

Als Rhamnolipide werden α-L-Rhamnopyranosyl-β-hydroxidecanonyl-β-hydroxidecansäure und 2-0-L Rhamnopyranosyl-α-L-rhamnopyranosyl-β-hydroxidecanoyl-β-hydroxidecansäure erhalten.

Aus diesem Stand der Technik ist ersichtlich, daß bisher nur die biotechnische Herstellung von Rhamnolipiden gelungen ist, die zwei Hydroxidecansäurereste neben einem oder zwei Rhamnoseresten enthalten.

Hieraus stellte sich die Aufgabe, weitere Rhamnolipide und Möglichkeiten zu finden, die auf die Zellmasse bezogene Ausbeute an Rhamnolipiden zu steigern.

Es wurde ein Verfahren zur Herstellung von Rhamnolipiden mit Mikroorganismen der Gattung Pseudomonas in einem wäßrigen, mindestens eine assimilierbare Kohlenstoffquelle enthaltenden Medium bei pH-Werten von über 4 und Temperaturen von 30° C und darüber gefunden.

Danach wird als Mikroorganismus ein aus einer Wasserprobe isolierter Stamm Pseudomonas species DSM 2874 bei einem pH-Wert von 6,5 bis 7,3 und einer Temperatur von 30 bis 37° C eingesetzt.

Die Zellen dieses Stammes Pseudomonas spec. DSM 2874 sind stäbchenförmig und haben bei einem Durchmesser von etwa 0,5 µm eine Länge von etwa 1,0 µm, sie sind beweglich und monopolar mit einer Geißel begeißelt. Die Kolonie dieses Pseudomonas spec. DSM 2874 ist auf dem Komplexmedium Hexadecan gelb gefärbt, hat einen glatten Kolonierand bei glänzender und undurchsichtiger Oberfläche. Der Durchmesser der Kolonie beträgt nach einem Tag etwa 1 mm. Der Stamm dieses Pseudomonas species DSM 2874 ist gramnegativ und aerob, seine Zelltrockenmasse hat eine beige Farbe. Dieser Stamm verflüssigt Gelatine und wächst bei einer Temperatur von 42°C auf Glucose. Er ist ferner hämolyse- und ureasepositiv. Es wurde weiter festgestellt, daß dieser Stamm folgende Stickstoff- bzw. Kohlenstoffquellen zu verwerten vermag:

<u>Stickstoffquellen</u>:
Ammoniumsulfat
Natriumnitrat bzw. -nitrit
Harnstoff.

<u>Kohlenstoffquellen</u>:

| | |
|---|---|
| Glucose | |
| Arabinose | Glutamat |
| Fructose | Serin |
| | Glycerin |
| Citrat | |
| Malonat | |
| Succinat | |
| Malat | |
| Pyruvat | Glycerin |
| | Äthanol |
| | Propanol |
| | Octanol |
| | Stearinsäure |
| | Myristinsäure |
| | Alkane |

Auch der erfindungsgemäß einzusetzende Stamm Pseudomonas spec. DSM 2874 bildet während aerober Züchtung in einer Nährstofflösung, deren pH-Wert während der Züchtung auf 6,5 bis 7,3 konstant gehalten wird, und zwar durch Zusatz entsprechender Mengen an Ammonium- oder Alkalihydroxidlösung, insbesondere Natriumhydroxidlösung, Rhamnolipide. Eine solche Nährlösung kann beispielsweise folgende Substanzen in den angegebenen g-Mengen in 1000 cm³ deionisiertem Wasser enthalten:

| | |
|---|---|
| $Na_2HPO_4 \cdot 2\,H_2O$ | 5,34 |
| $KH_2PO_4$ | 2,72 |

| | |
|---|---|
| $(NH_4)_2 SO_4 \cdot 7 H_2O$ | 8,0 |
| $CaCl_2 \cdot 2 H_2O$ | 0,2 |
| $MgSO_4 \cdot 7 H_2O$ | 0,4 |
| $FeSO_4 \cdot 7 H_2O$ | 0,02 |
| $MnSO_4 \cdot H_2O$ | 0,0025 |
| $NH_4$-Heptamolybdat | 0,001 |

Sie wird zunächst in herkömmlicher Weise sterilisiert und abgekühlt, bevor ihr die ebenfalls bereits sterilisierte, assimilierbare Kohlenstoffquelle zugesetzt wird. Als Kohlenstoffquelle wird vorzugsweise n-Paraffin mit Gehalten an Molekülen mit 14 C-Atomen von 89 % und mit 15 C-Atomen von 9 % eingesetzt. Selbstverständlich sind auch andere der vorstehend tabellarisch aufgelisteten Verbindungen als Kohlenstoffquelle mit ebenfalls gutem Erfolg einsetzbar.

Nach beendeter Züchtung wird die erhaltene Submerskultur in an sich bekannter Weise mit einem geeigneten Lösungsmittel, wie beispielsweise Äthylacetat extrahiert.

Die gewonnenen Extrakte, die die Rhamnolipide enthalten, werden vermischt und, vorzugsweise unter Vakuum zu einem Rückstand eingeengt, der letztlich in an sich bekannter Weise, vorzugsweise chromatographisch, aufgearbeitet wird.

Bei einer derartigen Züchtung werden mit Pseudomonas spec. DSM 2874 ebenfalls die bekannten Rhamnolipide 2-0-α-L-Rhamnopyranosyl-α-L-rhamnopyranosyl-β-hydroxidecanoyl-β-hydroxidecansäure

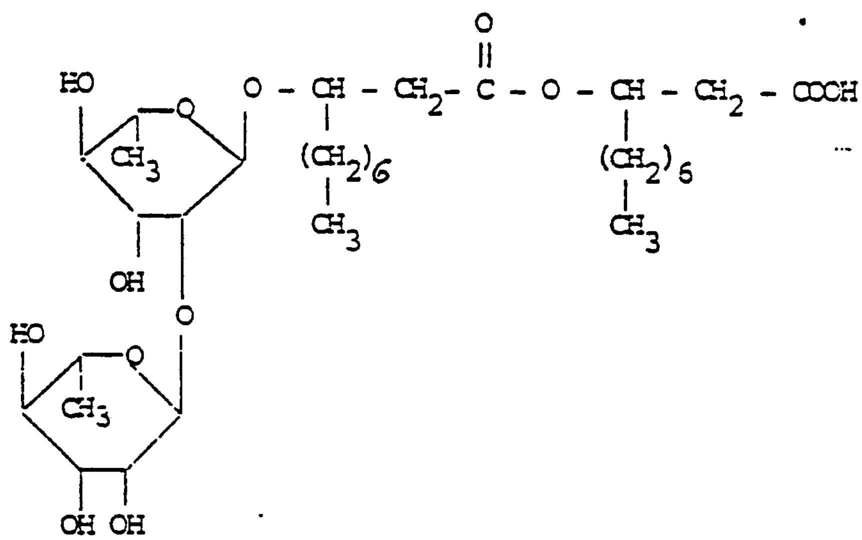

(I)

und α-L-Rhamnopyranosyl-β-hydroxidecanoyl-β-hydroxidecansäure

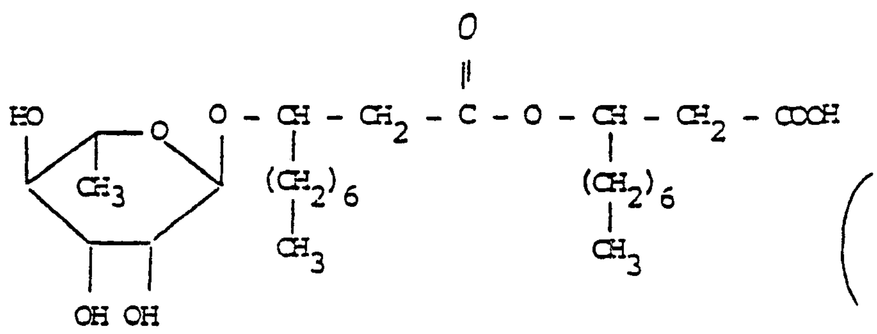

(II)

Die Ausbeuten aus der Summe dieser beiden Rhamnolipide liegen bei 0,1 g pro g Zelltrockenmasse bzw. bei 0,8 g/1000 $cm^3$ Kulturbrühe.

Wird Pseudomonas spec. DSM 2874 in gleicher Weise, aber unter Limitierung des Stickstoffangebots gezüchtet, so werden ebenfalls die angegebenen Rhamnolipide (I) und (II) erhalten, aber in Mengen von 0,5 g pro g Zelltrockenmasse bzw. etwa 12 g/1000 $cm^3$ Kulturbrühe. In diesem Fall wird die pH-Wert Einstellung während der Züchtung vorteilhaft mit Natriumhydroxidlösung vorgenommen.

Eine ähnliche Wirkung wird erreicht, wenn die Züchtung des Pseudomonas spec. DSM 2874 unter Limitierung der $Mg^{2+}$-Ionenkonzentration erfolgt. Die Ausbeute an Rhamnolipiden (I) und (II) beträgt etwa 0,4 g pro g Zelltrockenmasse.

Bezogen auf die Zelltrockenmasse kann die Ausbeute an Rhamnolipiden noch weiter gesteigert werden, wenn Pseudomonas spec. DSM 2874 unter aeroben Bedingungen in einer eine assimilierbare Kohlenstoffquelle enthaltende Nährlösung gezüchtet und anschließend von der Kulturbrühe die Zellfeuchtmasse abgetrennt wird. Aus der bei dieser Abtrennung hinterbleibenden flüssigen Phase können - wie vorstehend angegeben - Rhamnolipide isoliert werden.

Die gewonnene Zellfeuchtmasse (ruhende Zellen) wird, gegebenenfalls - auch nach einer Zwischenlagerung bei einer Temperatur von -30 bis -35°C -, in einer verdünnten Natriumchloridlösung, die 0,4 bis 0,6 Gew.-% NaCl enthält, suspendiert. Nach Zusatz einer assimilierbaren Kohlenstoffquelle, wie beispielsweise Glycerin oder n-Paraffin (89 % $C_{14}$ und 9 % $C_{15}$) wird diese Suspension unter lebhafter Bewegung bei einer Temperatur von etwa 30 bis 40°C inkubiert, wobei der pH-Wert der Suspension ständig überwacht und durch Zusatz entsprechender Mengen an 1 N Natriumhydroxidlösung auf einen Wert zwischen 6,0 und 6,8 gehalten wird. Nach beendeter Inkubation wird die Zellfeuchtmasse abgetrennt. Der pH-Wert des wäßrigen Überstandes wird mit der verdünnten, vorzugsweise 10 %-igen Lösung einer starken Mineralsäure, wie beispielsweise Schwefelsäure, Chlorwasserstoffsäure, auf etwa 2 abgesenkt und danach werden aus diesem die Rhamnolipide isoliert. Die Zellfeuchtmasse wird abermals in Natriumchloridlösung suspendiert und nach Zusatz einer assimilierbaren Kohlenstoffquelle in gleicher Weise, wie vorstehend beschrieben, inkubiert. Diese Vorgänge können mit den ruhenden Zellen noch einmal wiederholt werden. Danach ist die Produktionsaktivität dieser ruhenden Zellen praktisch Null.

Im ersten Einsatz der ruhenden Zellen ergibt sich eine Ausbeute von etwa 1,1 g Rhamnolipide pro g Zelltrockenmasse; beim zweiten Einsatz werden 32 % und beim dritten Einsatz noch 14 % dieser Menge an Rhamnolipiden als Ausbeute erhalten, so daß die Gesamtausbeute hierbei etwa 1,6 g Rhamnolipide pro g Zelltrockenmasse beträgt.

Hierbei wurden erstmals Rhamnolipide isoliert, die nur einen β-Hydroxidecanoyl-Rest im Molekül enthalten und Molekulargewichte von 334 bzw. 480 aufweisen. Diese Rhamnolipide haben die Strukturformeln

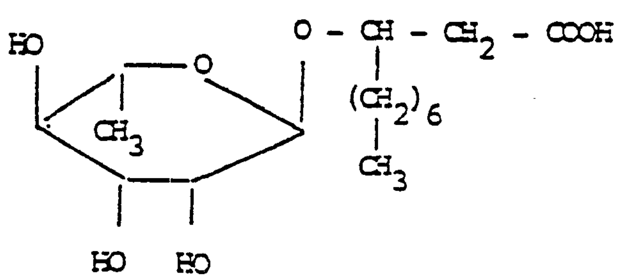

III

und

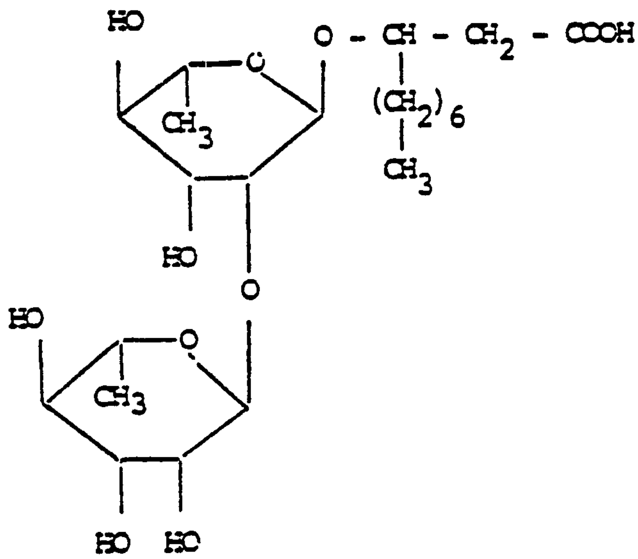

IV

und sind als α-L-Rhamnopyranosyl-β-hydroxidecansäure (Rhamnolipid (III)) bzw. 2-0-α-L Rhamnopyranosyl-α-L-rhamnopyranosyl-β-hydroxidecansäure (Rhamnolipid (IV)) zu bezeichnen. Diese Strukturen wurden eindeutig sichergestellt durch Elementaranalysen, $^{13}C$- und $^{1}H$-Kernresonanzspektroskopie und Massenspektroskopie.

Es ist weiter festgestellt worden, daß die Zusammensetzung der mit ruhenden Zellen erzeugten Gemische der Rhamnolipide (I) bis (IV) von der assimilierbaren Kohlenstoffquelle und bei gleicher Kohlenstoffquelle von der Inkubationstemperatur abhängig ist, wie nachfolgende Tabellen zeigen:

**Tabelle 1**

Abhängigkeit der Zusammensetzung eines mit ruhenden Zellen bei einer Inkubationstemperarur von 30°C erzeugten Gemisches der Rhamnolipide (I) bis (IV) von der Art der assimilierbaren Kohlenstoffquelle

| Rhamnolipid | Glycerin | n-Paraffin (89 % $C_{14}$, 9 % C 15) |
|---|---|---|
| (I) | 61,5 % | 41 % |
| (II) | 22,4 % | 42 % |
| (III) | 15,1 % | 15 % |
| (IV) | 1,0 % | 2 % |

**Tabelle 2**

Abhängigkeit der Zusammensetzung eines mit ruhenden Zellen auf der gleichen Kohlenstoffquelle n-Paraffin (89 % $C_{14}$, 9 % $C_{15}$) Gemisches an Rhamnolipiden (I) bis (IV) von der Inkubationstemperatur

| Rhamnolipid | 30°C | 37°C |
|---|---|---|
| (I) | 41 % | 32 % |
| (II) | 42 % | 66 % |
| (III) | 15 % | 1 % |
| (IV) | 2 % | 1 % |

Eine andere Möglichkeit, Rhamnolipide in guten Ausbeuten mit Pseudomonas spec. DSM 2874 zu erhalten, bietet eine andere Variante der erfindungsgemäßen Arbeitsweise. Danach wird Pseudomonas spec. DSM 2874 in einer eine assimilierbare Kohlenstoffquelle, wie beispielsweise Glucose, n-Paraffin oder dgl., enthaltenden Nährlösung, vorzugsweise bei einer Temperatur von 30a bis 37°C, aerob gezüchtet, wobei in der Kulturbrühe durch Zugabe entsprechender Mengen verdünnter, vorzugsweise 10 %-iger Natriumhydroxidlösung der pH-Wert um den Neutralpunkt, vorzugsweise auf 6,8 bis 7,2, konstant gehalten wird. Nach beendeter Inkubation wird von der Kulturbrühe die Zellfeuchtmasse abgetrennt und in einer verdünnten, vorzugsweise 0,5 bis 1,0 %-igen Natriumchloridlösung suspendiert und in diese Suspension eine Natriumalginat-Lösung eingerührt. Dieses Gemisch wird anschließend unter Rühren in eine ungesättigte Calciumchloridlösung eingetropft und dieses Gemisch weiter gerührt bis sich Granula gebildet haben, deren Durchmesser im Durchschnitt etwa 1,5 mm beträgt. Diese Granula werden von der flüssigen Phase abgetrennt und in einer ungesättigten, vorzugsweise 0,5 bis 1,0 Gew.-% NaCl, enthaltenden, Natriumchloridlösung eingetragen, die geringe Mengen an Calciumchlorid gelöst und eine assimilierbare Kohlenstoffquelle, wie beispielsweise Glycerin, n-Paraffin (89 % $C_{14}$, 9 % $C_{15}$) oder dgl. enthält. Nach der Inkubation, die bei einer Temperatur von 30 bis 40°C und einem pH-Wert von 6,5 bis 6,9 durchgeführt wird, werden die Granula von der flüssigen Phase abgetrennt, aus der die Rhamnolipide durch Extraktion isoliert werden. Die abgetrennten Granula können noch mehrmals als Biokatalysator in gleicher Weise eingesetzt werden. Sie verlieren ihre Aktivität wesentlich langsamer als die vorerwähnten ruhenden Zellen. Die relative Aktivität der Granula beträgt - ausgehend vom ersten Einsatz mit 100 % - im zweiten Einsatz 90 %, im dritten 44 % und im vierten Einsatz noch 34 %.

Auch mit den in Form von Granula immobilisierten Zellmassen des Pseudomonas spec. DSM 2874 werden die Rhamnolipide (I) bis (IV) erhalten.

Die erfindungsgemäß erzeugten Rhamnolipide sowie deren Mischungen zeichnen sich durch hervorragende grenzflächenaktive Wirkungen aus, die im System n-Hexadecan / Wasser in einem Temperaturbereich von 20 bis 90°C keine Temperaturabhängigkeit zeigt, durch pH-Wert-Änderungen im Bereich 3 bis 9 ebenso wenig beeinflußt wird, wie durch eine Salinität der wäßrigen Phase im Bereich zwischen > 0 und 14 Gew.-%. Eine Hydrolyse der Rhamnolipide konnte in diesem Rahmen ebenfalls nicht festgestellt werden.

Aus diesen Gründen eignen sich die erfindungsgemäß hergestellten Rhamnolipide vorzüglich als grenzflächenaktives Zusatzmittel für das tertiäre Fluten von Erdöllagerstätten. Versuche haben gezeigt, daß mit einer Zusatzmenge an Rhamnolipiden von nur 0,5 bis 0,7 g/l Flutwasser Mehrentölungen von über 12 % erreicht werden.

Der in dieser Anmeldung erwähnte Mikroorganismus Pseudomonas spec. wurde am 03.02.1984 bei der Deutschen Sammelstelle von Mikroorganismen, D-3400 Göttingen, Grisebachstr. 8, hinterlegt und hat das Aktenzeichen DSM 2874 erhalten.

**Beispiel 1**

**Bildung von Rhamnolipiden mit wachsenden Zellen**

Die Züchtung von Pseudomonas species DSM 2874 erfolgt bei 100 Upm und pH 6,8 in 500 ml Erlenmeyerkolben mit Schikanen, die jeweils 100 ml Kulturmedium enthalten (Zusammensetzung: 0,534 g $Na_2HPO_4 \cdot 2\ H_2O$, 0,272 g $KH_2PO_4$, 0,8 g $(NH_2)_2SO_4 \cdot 7\ H_2O$, 0,02 g $CaCl_2 \cdot 2\ H_2O$, 0,04 g $MgSO_4$, 0,002 g $FeSO_4 \cdot 7\ H_2O$, 0,00025 g $MnSO_4 \cdot H_2O$, 0,0001 g $NH_4$-Heptamolybdat und 100 ml dest. Wasser), das 30 min bei 121°C sterilisiert, nach Abkühlung auf 30°C mit 2 g sterilem Paraffin S (89 % $C_{14}$, 9 % $C_{15}$) versetzt und mit 1 ml einer 24 Std. alten Submerskultur von Pseudomonas spec. DSM 2874 beimpft wird. Während der Züchtung wird der pH-Wert der Submerskultur nach 24 h und 48 h mit steriler 1 N NaOH-Lösung auf pH 6,8 korrigiert. Die Züchtung ist nach 72 h beendet, danach werden in bekannter Weise die erhaltenen Submerskulturen erschöpfend mit Äthylacetat extrahiert. Die organischen Extrakte enthalten nach Konzentration im Vakuum 80 mg Rhamnolipide, und zwar 40 mg Rhamnolipid I = 50 %, bezogen auf Gesamtrhamnolipid, und 40 mg Rhamnolipid II = 50 %, bezogen auf Gesamtrhamnolipid.

**Beispiel 2**

**Bildung von Rhamnolipiden mit wachsenden Zellen unter Limitierung der $Mg^{2+}$-Ionen**

Pseudomonas species DSM 2874 wird, wie in Beispiel 1, gezüchtet jedoch sind anstelle von 0,04 g nur 0,002 g $MgSO_4 \cdot 7\ H_2O$ im Kulturmedium enthalten. Die Extraktion wird wie in Beispiel 1 durchgeführt. Die erhaltenen organischen Extrakte enthalten 113 mg Rhamnolipid II = 65 %. bezogen auf Gesamtrhamnolipid und 60 mg Rhamnolipid I = 35 %, bezogen auf Gesamtrhamnolipid.

**Beispiel 3**

**Bildung von Rhamnolipiden mit wachsenden Zellen unter Stickstoff-Limitierung**

Ein Bioreaktor, ausgerüstet mit einem Umwurfsystem, wird mit 20 l Nährsalzlösung (Zusammensetzung: 6 g Citrat-1-Hydrat, 88,4 g $Na_2HPO_4 \cdot 2\ H_2O$, 68 g $KH_2PO_4$, 160 g $(NH_4)_2SO_4$, 12 g $CaCl_2 \cdot 2\ H_2O$, 12 g $MgSO_4 \cdot 7\ H_2O$, 1,2 g $FeSO_4 \cdot 7\ H_2O$, 0,15 g $MgSO_4 \cdot H_2O$ und 0,036 g $NH_4$-Heptamolybdat und 20 l deionisiertes Wasser) versetzt, Phosphat getrennt in 1000 ml $H_2O$deion. sterilisieren und erst nach dem Sterilisieren zugeben, bei pH 3,0 und 121°C 30 min sterilisiert, nach Abkühlung auf 30°C mit 1.600 g sterilem Paraffin S (89 % $C_{14}$, 9 % $C_{15}$) versetzt. Der pH-Wert der Kulturlösung wird mit 10 %-iger $NH_4OH$-Lösung auf pH 6,6 eingestellt und mit 2000 ml einer 24 Std. alten Submerskultur von Pseudomonas species DSM 2874 beimpft. Während der Züchtung wird die Submerskultur automatisch mit einer pH-Regulierstation zunächst durch Titration mit 800 ml einer 10 %-igen $NH_4OH$-Lösung und im Anschluß daran durch Titration mit einer 10 %-igen NaOH-Lösung zwischen pH 6,6 und pH 7,2 konstant gehalten und bei 30°C, einer Umdrehungszahl von 1000 Upm und einer Belüftungsrate von 0,62 V/V/m mit Luft begast. Die Züchtung ist nach 168 Std. beendet, danach wird in bekannter Weise die erhaltene Submerskultur erschöpfend mit Äthylacetat extrahiert. Die vereinigten organischen Extrakte werden im Vakuum konzentriert, der org. Rohextrakt enthält neben Paraffin S (200 g), das recyclisiert wird, 156 g Rhamnolipid II = 65 %, bezogen auf Gesamtrhamnolipid und 83 g Rhamnolipid I = 35 %, bezogen auf Gesamtrhamnolipid.

**Beispiel 4**

**Züchtung von Pseudomonas species DSM 2874**

Ein Bioreaktor, ausgerüstet mit einem Umwurfsystem, wird mit 22 l einer Nährsalzlösung (Zusammensetzung: 5 g Citrat-1-Hydrat, 110 g $Na_2HPO_4 \cdot 2\ H_2O$, 85 g $KH_2PO_4$, 152,5 g $(NH_4)_2SO_4$, 10 g $CaCl_2 \cdot 2\ H_2O$, 10 g $MgSO_4 \cdot 7\ H_2O$, 1 g $FeSO_4 \cdot 7\ H_2O$, 0,125 g $MnSO_4 \cdot H_2O$ und 0,03 g $NH_4$-Heptamolybdat und 22 l deionisiertes Wasser) versetzt, bei pH 3,0 und 121°C 30 min. sterilisiert, nach Abkühlung auf 30°C mit 1000 g 50 gew.-%-iger steriler Glucoselösung versetzt, der pH-Wert mit 10 %-iger NaOH-Lösung auf pH 6,8 eingestellt und mit 2500 ml einer 24 h alten Submerskultur von Pseudomonas species DSM 2874 beimpft. Während der Züchtung wird die Submerskultur automatisch mit einer pH-Regulierstation durch Titration mit einer 10 %-igen NaOH-Lösung zwischen pH 6,8 und pH 7,2 konstant gehalten und bei 30°C, einer Umdrehungszahl von 1500 Upm und einer Belüftungsrate von 0,62 V/V/m mit Luft begast. Die Glucose ist nach 9 Std. verbraucht, danach wird die Zellmasse aus der Kultursuspension in bekannter Weise durch Zentrifugation abgetrennt. Es werden 0,92 kg Zellfeuchtmasse erhalten und diese bei -32°C gelagert.

**Beispiel 5**

**Bildung vom Rhamnolipiden mit ruhenden Zellen**

20 g der nach Beispiel 4 gewonnenem Zellfeuchtmasse werden in 100 ml 0,5 gew.-%-iger NaCl-Lösung suspendiert, mit 4 g Glyzerin versetzt und diese Suspension bei 30°C und 100 Upm in einem 500 ml Erlenmeyerkolben auf einer Rotationsschüttelmaschine inkubiert. Der pH-Wert wird mit 1 N NaOH-Lösung auf pH 6,6 korrigiert. Nach 168 Std. Inkubationszeit wird die Suspension mit 10 gew.-%-iger $H_2SO_4$ auf pH 2,0 eingestellt und erschöpfend mit Äthylacetat extrahiert. Die vereinigten organischen Extrakte werden im Vakuum konzentriert. Der organische Rohextrakt enthält:

| | |
|---|---|
| 430,0 mg Rhamnolipid (I) | entsprechend 61,5 % |
| 156,8 mg Rhamnolipid (II) | entsprechend 22,4 % |
| 105,7 mg Rhamnolipid (III) | entsprechend 15,1; % und |
| 7 mg Rhamnolipid (IV) | entsprechend 1 %. |

Die Prozentzahlen bezeichnen den prozentualen Anteil des einzelnen Rhamnolipids an der erhaltenen Rhamnolipid-Gesamtmenge.

**Beispiel 6**

**Bildung von Rhamnolipiden mit ruhenden Zellen**

20 g der nach Beispiel 4 gewonnenen Zellfeuchtmasse werden in 100 ml 0,5 gew.-%-iger NaCl-Lösung suspendiert, mit 4 g n-Paraffin (89 % $C_{14}$, 9 % $C_{15}$) versetzt und wie in Beispiel 5 beschrieben, inkubiert und extrahiert. Der organische Rohextrakt enthält:

| | |
|---|---|
| 639,6 mg Rhamnolipid (I) | entsprechend 41 % |
| 655,2 mg Rhamnolipid (II) | entsprechend 42 % |
| 234 mg Rhamnolipid (III) | entsprechend 15 % |
| 31,2 mg Rhamnolipid (IV) | entsprechend 2 % |

jeweils bezogen auf die Gesamtmenge der Ausbeute an Rhamnolipiden.

**Beispiel 7**

**Bildung von Rhamnolipiden mit ruhenden Zellen**

20 g der nach Beispiel 4 gewonnenen Zellfeuchtmasse werden in 100 ml 0,5 gew.-%-iger NaCl-Lösung suspendiert, mit 4 g n-Paraffin (89 % $C_{14}$, 9 % $C_{15}$) versetzt und wie in Beispiel 5 beschrieben, jedoch bei 37°C inkubiert und anschließend extrahiert. Der organische Rohextrakt enthält 450 mg Rhamnolipid I = 62 %, bezogen auf Gesamtrhamnolipid und 270 mg Rhamnolipid II = 37 %, bezogen auf Gesamtrahmnolipid. Die Rhamnolipide III und IV sind $<1$ %, bezogen auf Gesamtrhamnolipid.

**Beispiel 8**

**Bildung von Rhamnolipiden mit immobilisierten Zellen**

40 g der nach Beispiel 4 gewonnenen Zellfeuchtmasse werden in 40 ml 0,9 gew.-%-iger NaCl-Lösung suspendiert und diese Suspension wird unter Rührung in 360 ml einer wäßrigen 3 gew.-%-igen Na-Alginat-Lösung eingetragen. Die so erhaltene Suspension wird unter Rührung in 600 ml 2 gew.-%-iger $CaCl_2$-Lösung eingetropft. Die ionotrope Vernetzung ist nach 40 min. beendet und die Granula mit einem mittleren Durchmesser von 1,5 mm werden abfiltriert. Die so erhaltenen immobilisierten Zellen werden in 1000 ml einer wäßrigen Lösung, die 2 Gew.-% Glyzerin, 0,25 Gew.-% NaCl und 0,037 Gew.-% $CaCl_2$ enthält, 70 Std. bei 30°C und pH 6,8 inkubiert. Die immobilisierten Zellen werden abfiltriert und können erneut als Biokatalysator eingesetzt werden. Das wäßrige Filtrat wird wie in Beispiel 5 extrahiert. Der organische Rohextrakt enthält 1830 mg Rhamnolipid I = 61 %, bezogen auf Gesamtrhamnolipid, 630 mg Rhamnolipid II = 21 %, bezogen auf Gesamtrhamnolipid, 480 mg Rhamnolipid III = 16 %, bezogen auf Gesamtrhamnolipid und 60 mg Rhamnolipid IV = 2 %, bezogen auf Gesamtrhamnolipid.

**Patentansprüche**

1. Verfahren zur Herstellung von Rhamnolipiden mit Mikroorganismen der Gattung Pseudomonas in einem wäßrigen, mindestens eine assimilierbare Kohlenstoffquelle enthaltenden Medium bei pH-Werten über 4 und Temperaturen von 30°C und darüber, dadurch gekennzeichnet, daß als Mikroorgamismus ein aus einer Wasserprobe isolierter Stamm Pseudomonas species DSM 2874 bei einem pH-Wert von 6,7 bis 7,3 und einer Temperatur von 30 bis 37°C eingesetzt wird.

2. Verfahren nach Anspruch 1, dadurch gekennzeichnet, daß Pseudomonas species DSM 2874 unter aeroben Bedingungen in einer Nährstofflösung, die eine assimilierbare Kohlenstoffquelle enthält und in ihrem Stickstoffgehalt limitiert ist, gezüchtet wird und anschließend aus der Submerskultur die Rhamnolipide isoliert werden.

3. Verfahren nach Anspruch 1, dadurch gekennzeichnet, daß Pseudomonas spec. DSM 2874 unter aeroben Bedingungen in einer Nährstofflösung, die eine assimilierbare Kohlenstoffquelle enthält und in ihrem Gehalt an $Mg^{2+}$-Ionen limitiert ist, gezüchtet wird und anschließend aus der Submerskultur die Rhamnolipide isoliert werden.

4. Verfahren nach Anspruch 1, dadurch gekennzeichnet, daß Pseudomonas spec. DSM 2874 unter aeroben Bedingungen in einer eine assimilierbare Kohlenstoffquelle enthaltenden Nährlösung gezüchtet und anschliessend von der Kulturbrühe eine Zellfeuchtmasse abgetrennt, die in einer verdünnten Natriumchloridlösung suspendiert und nach Zusatz einer assimilierbaren Kohlenstoffquelle bei einer Temperatur von 30 bis 40° C unter Konstanthaltung des pH-Wertes auf 6,0 bis 6,8 inkubiert wird, worauf der pH-Wert der Suspension mit einer verdünnten Lösung einer starken Mineralsäure auf etwa 2,0 eingestellt wird und anschließend daraus die Rhamnolipide isoliert werden.

5. Verfahren nach Anspruch 4, dadurch gekennzeichnet, daß die aus der Kulturbrühe abgetrennte Zellfeuchtmasse vor ihrer Suspendierung in der verdünnten Natriumchloridlösung bei Temperaturen von -30 bis -35° C gelagert wird.

6. Verfahren nach Ansprüchen 4 bis 5, dadurch gekennzeichnet, daß aus der Suspension der Zellfeuchtmasse in Natriumchloridlösung im Anschluß an die Inkubation von der flüssigen Phase die Zellfeuchtmasse abgetrennt und in gleicher Weise abermals in Natriumchloridlösung suspendiert und inkubiert wird, während aus der flüssigen Phase die Rhamnolipide isoliert werden.

7. Verfahren nach Anspruch 1, dadurch gekennzeichnet, daß Pseudomonas spec. DSM 2874 unter aeroben Bedingungen in einer eine assimilierbare Kohlenstoffquelle enthaltenden Nährlösung gezüchtet und anschliessend von der Kulturbrühe eine Zellfeuchtmasse abgetrennt, in einer verdünnten Natriumchloridlösung suspendiert und in diese Suspension eine Natriumalginatlösung eingerührt wird, worauf die Suspension unter Rühren in eine ungesättigte Calciumchloridlösung eingetropft und weiter gerührt wird, bis sich Granula gebildet haben, die von der flüssigen Phase abgetrennt und in eine ungesättigte, geringe Menge Calciumchlorid und eine assimilierbare Kohlenstoffquelle enthaltende Natriumchloridlösung bei einer Temperatur von 30 bis 40° C und unter Aufrechterhaltung eines pH-Wertes von 6,5 bis 6,9 inkubiert werden, worauf die Granula von der flüssigen Phase abgetrennt werden, aus der die Rhamnolipide isoliert werden.

8. Verfahren nach Anspruch 7, dadurch gekennzeichnet, daß die nach der Inkubation von der flüssigen Phase abgetrennten Granula erneut inkubiert werden.

9. Rhamnolipide dadurch gekennzeichnet, daß sie nur einen β-hydroxidecanoyl-Rest im Molekül und Molekulargewichte von 334 bzw. 480 aufweisen.

## Claims

1. A process for the preparation of rhamnolipids using microorganisms of the genus Pseudomonas in an aqueous medium containing one or more assimilable carbon sources, at a pH greater than 4 and at a temperature of 30° C or higher, wherein a strain Pseudomonas species DSM 2874 isolated from a water sample is used as the microorganism, at a pH of from 6.7 to 7.3 and at from 30 to 37° C.

2. A process as claimed in claim 1, wherein Pseudomonas species DSM 2874 is cultivated under aerobic conditions in a nutrient solution which contains an assimilable carbon source and is limited in its nitrogen content, and the rhamnolipids are then isolated from the submerse culture.

3. A process as claimed in claim 1, wherein Pseudomonas spec. DSM 2874 is cultivated under aerobic conditions in a nutrient solution which contains an assimilable carbon source and is limited in its content of $Mg^{2+}$ ions, and the rhamnolipids are then isolated from the submerse culture.

4. A process as claimed in claim 1, wherein Pseudomonas spec. DSM 2874 is cultivated under aerobic conditions in a nutrient solution containing an assimilable carbon source, and a moist cellular material is then isolated from the culture broth, suspended in a dilute sodium chloride solution and, after the addition of an assimilable carbon source, incubated at from 30 to 40° C while keeping the pH constant at 6.0 - 6.8, after which the pH of the suspension is brought to about 2.0 with a dilute solution of a strong mineral acid and the rhamnolipids are then isolated therefrom.

5. A process as claimed in claim 4, wherein the moist cellular material isolated from the culture broth is stored at from -30 to -35° C before being suspended in the dilute sodium chloride solution.

6. A process as claimed in claims 4 and 5, wherein the moist cellular material is isolated from the liquid phase of the suspension of the moist cellular material in sodium chloride solution after the incubation and is suspended in sodium chloride solution and incubated repeatedly in the same manner, while the rhamnolipids are isolated from the liquid phase.

7. A process as claimed in claim 1, wherein Pseudomonas spec. DSM 2874 is cultivated under aerobic conditions in a nutrient solution containing an assimilable carbon source, and a moist cellular material is then isolated from the culture broth and suspended in a dilute sodium chloride solution and a sodium alginate solution is stirred into this suspension, after which the suspension is added dropwise to an unsaturated calcium chloride solution while stirring, and stirring is continued until granula have formed, the said granula being isolated from the liquid phase and being incubated in an unsaturated sodium chloride solution containing a small amount of calcium chloride and an assimilable carbon source, at from 30 to 40° C and while maintaining a

pH of from 6.5 to 6.9, after which the granula are separated off from the liquid phase, from which the rhamnolipids are isolated.

8. A process as claimed in claim 7, wherein the granula separated off from the liquid phase after the incubation are incubated again.

9. A rhamnolipid which has only one β-hydroxydecanoyl radical in the molecule and a molecular weight of 334 or 480.

**Revendications**

1. Procédé de préparation de rhamnolipides avec des microorganismes de l'espèce Pseudomonas, dans un milieu aqueux, contenant au moins une source de carbone assimilable, à des valeurs de pH supérieures à 4 et des températures de 30° C et au-dessus, caractérisé par le fait que l'on applique, comme microorganismes, une souche, isolée d'un prélèvement d'eau, de Pseudomonas species DSM 2874, à un pH de 6,7 à 7,3 et une température de 30 à 37° C.

2. Procédé selon la revendication 1, caractérisé par le fait que l'on cultive Pseudomonas species DSM 2874 sous conditions aérobies dans une solution nutritive qui contient une source de carbone assimilable et est limitée en teneur d'azote, et on isole ensuite les rhamnolipides de la culture immergée.

3. Procédé selon la revendication 1, caractérisé par le fait que l'on cultive Pseudomonas species DSM 2874 sous conditions aérobies dans une solution nutritive qui contient une source de carbone assimilable et est limitée en teneur d'ion $Mg^{2+}$ et on isole ensuite les rhamnolipides de la culture immergée.

4. Procédé selon la revendication 1, caractérisé par le fait que l'on cultive Pseudomonas species DSM 2874 sous conditions aérobies dans une solution nutritive qui contient une source de carbone assimilable, puisqu'on sépare du bouillon de culture une masse imprégnée de cellules que l'on susprend dans une solution diluée de chlorure de sodium et, après addition d'une source de carbone assimilable est incubée à une température de 30 à 40° C en maintenant constant le pH à une valeur de 6,0 à 6,8, après quoi on règle à environ 2,0 le pH de la suspension avec une solution diluée d'un acide minéral fort, puis on isole les rhamnolipides.

5. Procédé selon la revendication 4, caractérisé par le fait que la masse imprégnée de cellules séparée du bouillon de culture, est entreposée à des températures de -30 à -35° C avant d'être mise en suspension dans la solution de chlorure de sodium.

6. Procédé selon les revendications 4 à 5, caractérisé par le fait que, de la suspension de la masse imprégnée de cellules dans la solution de chlorure de sodium, à la suite de l'incubation, on sépare de la phase liquide la masse imprégnée de cellules et on la suspend et l'incube de la même manière de nouveau dans une solution de chlorure de sodium, tandis qu'on isole les rhamnolipides de la phase liquide.

7. Procédé selon la revendication 1, caractérisé par le fait que l'on cultive Pseudomonas species DSM 2874 sous conditions aérobies dans une solution nutritive qui contient une source de carbone assimilable, puisqu'on sépare du bouillon de culture une masse imprégnée de cellules que l'on suspend dans une solution diluée de chlorure de sodium et, dans cette suspension, on délaye une solution d'alginate de sodium, après quoi on introduit goutte à goutte, en agitant, la suspension dans une solution de chlorure de calcium insaturée et on continue à agiter jusqu'à ce que se soient formés des granules qui sont séparés de la phase liquide et qui sont incubés à une température de 30 à 40° C en maintenant constant le pH à une valeur de 6,0 à 6,8, dans une solution de chlorure de sodium insaturée, contenant de faibles quantités de chlorure de calcium et une source de carbone assimilable, après quoi on sépare de la phase liquide les granules desquels on isole les rhamnolipides.

8. Procédé selon la revendication 7, caractérisé par le fait que les granules séparés, après l'incubation, de la phase liquide sont à nouveau incubés.

9. Rhamnolipides caractérisés par le fait qu'ils possèdent seulement un reste β-hydroxydecanoyle dans la molécule et des poids moléculaires de 334 et 480.